(19) Europäisches Patentamt / European Patent Office / Office européen des brevets

(11) **EP 1 980 390 B2**

(12) **NEW EUROPEAN PATENT SPECIFICATION**
After opposition procedure

(45) Date of publication and mention
of the opposition decision:
**07.12.2016 Bulletin 2016/49**

(45) Mention of the grant of the patent:
**19.06.2013 Bulletin 2013/25**

(21) Application number: **07707661.0**

(22) Date of filing: **30.01.2007**

(51) Int Cl.:
*B32B 5/26* *(2006.01)*  *D04H 3/16* *(2006.01)*
*D04H 1/54* *(2012.01)*  *A61F 13/15* *(2006.01)*
*A61F 13/49* *(2006.01)*  *A61F 13/514* *(2006.01)*
*B32B 27/32* *(2006.01)*  *D04H 13/00* *(2006.01)*
*B32B 27/02* *(2006.01)*  *B32B 27/12* *(2006.01)*

(86) International application number:
**PCT/JP2007/051430**

(87) International publication number:
**WO 2007/088828 (09.08.2007 Gazette 2007/32)**

(54) **NONWOVEN-FABRIC LAMINATE, MOISTURE-PERMEABLE NONWOVEN-FABRIC LAMINATED SHEET COMPRISING NONWOVEN-FABRIC LAMINATE, AND SANITARY SUPPLY EMPLOYING THESE**

VLIESSTOFFLAMINAT, FEUCHTIGKEITSDURCHLÄSSIGES LAMINIERTES VLIESSTOFFFLÄCHENGEBILDE MIT DEM VLIESSTOFFLAMINAT UND DIESE VERWENDENDER SANITÄRER VORRAT

STRATIFIE DE NONTISSES, FEUILLE STRATIFIEE PERMEABLE A L'HUMIDITE COMPRENANT LE STRATIFIE DE NONTISSES ET PRODUIT SANITAIRE LES EMPLOYANT

(84) Designated Contracting States:
**AT BE BG CH CY CZ DE DK EE ES FI FR GB GR HU IE IS IT LI LT LU LV MC NL PL PT RO SE SI SK TR**

(30) Priority: **03.02.2006 JP 2006027188**

(43) Date of publication of application:
**15.10.2008 Bulletin 2008/42**

(73) Proprietor: **Mitsui Chemicals, Inc.**
**Tokyo 105-7117 (JP)**

(72) Inventor: **HISAMOTO, Takashi**
**Sodegaura-shi, Chiba 299-0265 (JP)**

(74) Representative: **Hoffmann Eitle**
**Patent- und Rechtsanwälte PartmbB**
**Arabellastraße 30**
**81925 München (DE)**

(56) References cited:
WO-A1-2005//080523    WO-A1-2005//095700
DE-A1- 10 212 842      JP-A- 2000 328 420
JP-A- 2001 234 459     JP-A- 2003 220 660
US-A- 3 849 241        US-A- 4 041 203
US-A- 5 939 341        US-A1- 2003 186 612
US-A1- 2004 058 609    US-A1- 2004 097 895
US-A1- 2005 124 240    US-A1- 2003/ 149 411
US-B1- 6 309 987       US-B1- 6 420 625
US-B2- 6 926 704

- Datasheet of sample for thickness measurement
- Optical method measurement protocol
- Delamination protocol
- Calender specification
- Catalogue Pegas 2005
- Declaration of Frantisek Klaska
- Declaration of Ivo E. Ruzek
- Article on INDEX 2005 Exhibition
- List of participants at INDEX 2005 Exhibition
- Datasheet regarding first novelty destroying sample
- Datasheet regarding second novelty destroying sample
- Datasheet regarding third novelty destroying sample
- Invoices regarding third novelty destroying sample
- Excerpt from www.pegasas.cz of 21-12-2003
- Excerpt from www.pegasas.cz of 05-02-2005
- Excerpt from www.pegasas.cz of 23-07-2005
- W. ALBRECHT ET AL.: 'Nonwoven Fabrics', 2003, WILEY-VCH VERLAG GMBH & CO, WEINHEIM pages 220 - 221
- W. ALBRECHT ET AL.: 'Nonwoven Fabrics', 2003, WILEY-VCH VERLAG GMBH & CO., WEINHEIM pages 364 - 368
- O. JIRSÁK, PROF. RNDR. ET AL.: 'Netkané textilie', 2003 pages 112 - 113
- Proof of publication of D20

EP 1 980 390 B2

**Description**

TECHNICAL FIELD

The present invention relates to a nonwoven fabric laminate which is excellent in softness, has water resistance and is inhibited from occurrence of a phenomenon that a hot melt adhesive penetrates through a nonwoven fabric layer and bleeds outside when a nonwoven fabric is laminated to another sheet material such as a moisture-permeable film using the hot melt adhesive, that is, so-called bleed-through, a moisture-permeable nonwoven fabric laminated sheet using the nonwoven fabric laminate, and sanitary products using them.

BACKGROUND ART

In sanitary products, such as disposable diapers, training pants and sanitary napkins, nonwoven fabrics have been used for liners, movable layers, backsheets, side gathers, etc. for many purposes in the past. Of these, a spunbonded (also referred to as "SB" hereinafter) nonwoven fabric composed of a propylene-based polymer, a so-called SMS nonwoven fabric in which a melt blown (also referred to as "MB" hereinafter) nonwoven fabric composed of a propylene-based polymer is interposed between SB nonwoven fabrics, etc. have been used most because they are lightweight and soft. The SMS nonwoven fabric, however, tends to become a little harder as compared with a single-layer SB nonwoven fabric though it has been improved in water resistance, and the above nonwoven fabrics have both merits and demerits.

In order to improve softness, water resistance, etc. of the SMS nonwoven fabric, a SMS nonwoven fabric which uses a polypropylene SB nonwoven fabric having a fineness of 0.7 to 1.5 dtex and a MB nonwovne fabric layer having a fiber diameter of 1 to 5 $\mu$m and which has been designed so that the basis weight of the MB nonwoven fabric may become not less than 1 g/m$^2$ and not more than 33% of the overall basis weight and the 5% modulus index may become not less than 0.41 has been proposed as the SMS nonwoven fabric (e.g., patent document 1).

The SMS nonwoven fabric having a 5% modulus of not less than 0.41, however, is still insufficient in softness, and a SMS nonwoven fabric in which the SB nonwoven fabrics laminated on both side surfaces of the MB nonwoven fabric have the same basis weight as each other becomes bulky and is still insufficient for sanitary products that have been desired to have softness and to be lightened in weight.

Further, when the MB nonwoven fabric or the SMS nonwoven fabric is used for sanitary products, a moisture-permeable film, a waterproof film or the like is sometimes bonded (laminated) thereto in order to further give a function, and as a means for the lamination, a hot melt adhesive has been used. Such a hot melt adhesive penetrates into the nonwoven fabric layer in the melting process, and therefore, prevention of bleeding of such a hot melt adhesive onto the opposite side of the nonwoven fabric layer is desired.

Patent document 1: Japanese Patent Laid-Open Publication No. 3096/2004

DISCLOSURE OF THE INVENTION

PROBLEM TO BE SOLVED BY THE INVENTION

It is an object of the present invention to provide a nonwoven fabric laminate which is inhibited from bleed-through of an adhesive when a SMS nonwoven fabric is laminated onto another sheet material with a hot melt adhesive or the like and which has excellent softness, water resistance and air permeability.

MEANS TO SOLVE THE PROBLEM

The present inventors have studied the aforesaid problem, and they have found that the above object can be attained by adjusting the basis weight of the SB nonwoven fabric (first SB nonwoven fabric) that is brought into contact with a hot melt adhesive or the like to not less than 1.6 times the basis weight of the SB nonwoven fabric (second SB nonwoven fabric) that is not brought into contact with the hot melt adhesive or the like, adjusting the thickness of the first SB nonwoven fabric to not less than 1.4 times the thickness of the second SB nonwoven fabric, and decreasing the basis weight of the MB nonwoven fabric.

That is to say, the present invention is a nonwoven fabric laminate which comprises a first spunbonded nonwoven fabric comprising a propylene-based polymer, a melt blown nonwoven fabric comprising a propylene-based polymer and laminated on the first spunbonded nonwoven fabric, and a second spunbonded nonwoven fabric comprising a propylene-based polymer and laminated on the melt blown nonwoven fabric,
wherein the first spun-bonded nonwoven fabric, the melt-blown nonwoven fabric and the second spun-bonded nonwoven fabric are laminated in this order;

the nonwoven fabric laminate having overall basis weight of not more than 30 g/m$^2$, wherein:

the basis weight of the first spunbonded nonwoven fabric is in the range of 3 to 25 g/m$^2$, the basis weight of the second spunbonded nonwoven fabric is in the range of 1 to 11 g/m$^2$, the ratio of the basis weight of the first spunbonded nonwoven fabric to the basis weight of the second spunbonded nonwoven fabric (basis weight of first spunbonded nonwoven fabric/basis weight of second spunbonded nonwoven fabric) is not less than 1.6, the ratio of a thickness of the first spunbonded nonwoven fabric to a thickness of the second spunbonded nonwoven fabric (thickness of first spunbonded nonwoven fabric/thickness of second spunbonded nonwoven fabric) is not less than 1.4, the basis weight of the melt blown nonwoven fabric is less than 3 g/m$^2$, and the compression bond area ratio is in the range of 6 to 25%.

In the nonwoven fabric laminate, it is preferable that fibers to form the first spunbonded nonwoven fabric and the second spunbonded nonwoven fabric have a fineness of not more than 1.56 dtex (1.4deniers) and fibers to form the melt blown nonwoven fabric have a fiber diameter of 1 to 5 $\mu$m.

The nonwoven fabric laminate preferably has a 5% modulus index of 0.15 to 0.38 N/30 mm/(g/m$^2$).

The present invention includes a moisture-permeable nonwoven fabric laminated sheet obtained by laminating a moisture-permeable film on a surface of the first spunbonded nonwoven fabric of the nonwoven fabric laminate through a hot melt adhesive.

In the moisture-permeable nonwoven fabric laminated sheet of the invention, the moisture-permeable film is preferably a porous film.

The present invention includes a disposable diaper having the moisture-permeable nonwoven fabric laminated sheet as a backsheet.

The present invention includes a sanitary napkin having the moisture-permeable nonwoven fabric laminated sheet as a backsheet.

The present invention includes a sanitary product comprising the nonwoven fabric laminate.

The present invention includes a sanitary product comprising the moisture-permeable nonwoven fabric laminated sheet.

The present invention includes a sanitary product comprising the nonwoven fabric laminate and the moisture-permeable nonwoven fabric laminated sheet.

The present invention includes a composite nonwoven fabric laminated sheet obtained by laminating at least one sheet material selected from the group consisting of a nonwoven fabric, a woven fabric, a paper, a waterproof sheet, a net and a synthetic resin sheet on a surface of the first spunbonded nonwoven fabric of the nonwoven fabric laminate through a hot melt adhesive.

The present invention includes a sanitary product comprising the composite nonwoven fabric laminated sheet.

The sanitary product comprising the composite nonwoven fabric laminated sheet may comprise the composite nonwoven fabric laminated sheet as a solid gather.

EFFECT OF THE INVENTION

In the nonwoven fabric laminate of the present invention, the first spunbonded nonwoven fabric on the side where a hot melt adhesive is to be applied is formed in large thickness, and when a hot melt adhesive layer having been applied to the surface of the first spunbonded nonwoven fabric is heated, most of the molten hot melt adhesive is diffused into voids of the first spunbonded nonwoven fabric and absorbed therein. On this account, the quantity of the hot melt adhesive that reaches the melt blown nonwoven fabric for blocking bleed-through of the hot melt adhesive is decreased, and bleed-through of the hot melt adhesive can be almost completely blocked by the melt blown nonwoven fabric. Therefore, the hot melt adhesive hardly reach the back surface side, that is, the surface of the second spunbonded nonwoven fabric.

Further, since the first spunbonded nonwoven fabric of large thickness is arranged, the quantity of the hot melt adhesive that reaches the melt blown nonwoven fabric is decreased, and the basis weight occupied by the melt blown nonwoven fabric required to block the bleed-through in the overall basis weight of the nonwoven fabric laminate can be decreased. Therefore, softness of the nonwoven fabric laminate is enhanced, and the nonwoven fabric laminate of the invention has a softer feeling in use as a whole than a conventional nonwoven fabric laminate having the same overall basis weight.

Accordingly, the nonwoven fabric laminate of the invention almost completely prevents bleed-through of a hot melt adhesive, and besides, it is very rich in softness.

BRIEF DESCRIPTION OF THE DRAWINGS

Fig. 1 is a schematic side view of a nonwoven fabric laminate of the present invention.
Fig. 2 is a schematic side view of a moisture-permeable nonwoven fabric laminated sheet of the present invention.
Fig. 3 is a schematic side view of an absorbing part of a disposable diaper that is a typical example of a sanitary product of the present invention.

Description of symbols

10: nonwoven fabric laminate
12: melt blown nonwoven fabric
14: first spunbonded nonwoven fabric
16: second spunbonded nowoven fabric
18: hot melt adhesive application predetermined surface
20: moisture-permeable nonwoven fabric laminated sheet
22: hot melt adhesive
24: moisture-permeable film
30: absorbing part of disposable diaper
32: top sheet
34: absorber

BEST MODE FOR CARRYING OUT THE INVENTION

Next, the nonwoven fabric laminate, the moisture-permeable nonwoven fabric laminated sheet and the sanitary products using them according to the invention are described in detail.

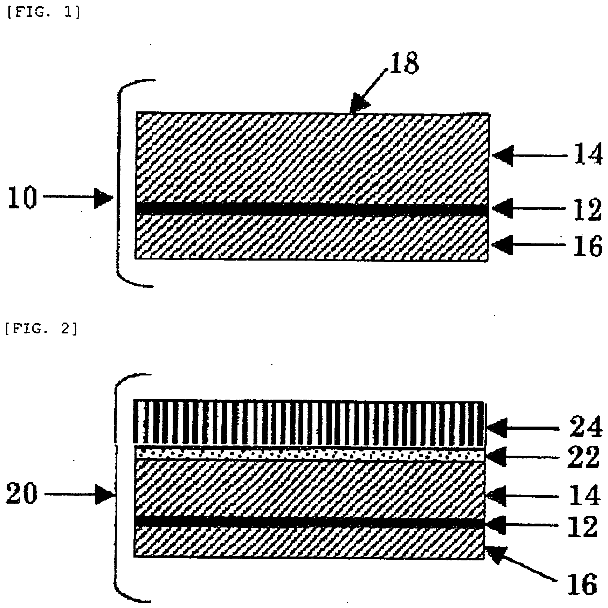

As shown in Fig. 1, the nonwoven fabric laminate 10 of the invention has a structure in which a first spunbonded nonwoven fabric (referred to as a "first SB nonwoven fabric" hereinafter) 14, a melt blown nonwoven fabric (referred to as a "MB nonwoven fabric" hereinafter) 12 and a second spunbonded nonwoven fabric (referred to as a "second SB nonwoven fabric" hereinafter) 16 are laminated in this order. The first SB nonwoven fabric 14 and the second SB nonwoven fabric 16 are each constituted of at least one spunbonded nonwoven fabric layer, and the MB nonwoven fabric 12 is constituted of at least one melt blown nonwoven fabric layer. In order to obtain the nonwoven fabric laminate 10 of the invention with optimum productivity, it is preferable that the first SB nonwoven fabric is constituted of two or more layers, the second SB nonwoven fabric is constituted of one layer, and the MB nonwoven fabric is constituted of one layer.

The surface of the first SB nonwoven fabric 14 is a hot melt adhesive application predetermined surface 18 where a hot melt adhesive is applied when a moisture-permeable nonwoven fabric laminated sheet 20 is produced using the nonwoven fabric laminate, as shown in Fig. 2.

In the present invention, the first SB nonwoven fabric 14 and the second SB nonwoven fabric 16 are each formed by melt spinning through a spunbonding method using the following propylene-based polymer.

The propylene-based polymer is specifically a homopolymer of propylene or a copolymer of propylene and another $\alpha$-olefin. Examples of the $\alpha$-olefins to be copolymerized include $\alpha$-olefins of 2 to 20 carbon atoms, such as ethylene, 1-butene, 1-pentene, 1-hexene, 1-octene, 1-decene, 3-methyl-1-butene, 3-methyl-1-pentene, 3-ethyl-1-pentene, 4-methyl-1-pentene and 4-methyl-1-hexene. Of these, ethylene and 1-butene are preferable, and ethylene is particularly preferable. Such $\alpha$-olefins may be copolymerized singly or in combination of two or more kinds. When a copolymer of propylene and another $\alpha$-olefin is used, the content of structural units derived from the $\alpha$-olefin in the copolymer is preferably not more than 5.0% by mol.

In the present invention, to the propylene-based polymer may be added another polymer, a colorant, a heat stabilizer, a nucleating agent and the like when needed, within limits not detrimental to the object of the invention.

Also for the MB nonwoven fabric 12, the propylene-based polymer described above is employable similarly to the first SB nonwoven fabric 14 and the second SB nonwoven fabric 16.

The structure of the nonwoven fabric laminate 10 of the invention is a structure wherein the MB nonwoven fabric 12 is interposed between the first SB nonwoven fabric 14 and the second SB nonwoven fabric 16 and they are partially compression bonded and united in one body, as shown in Fig. 1.

The propylene-based polymer for use in the first SB nonwoven fabric 14 and the second SB nonwoven fabric 16 to constitute the nonwoven fabric laminate 10 has a melt flow rate (measured at a temperature of 230°C under a load of 2.16 kg, also referred to as "MFR" hereinafter) of preferably 20 to 200 g/10 min, more preferably 50 to 150 g/10 min. The reason is that if the MFR is less than the lower limit of the above range, there is a fear that spinning becomes difficult when fibers of small fineness are obtained, and if the MFR is more than the upper limit of the above range, there is a fear that properties of the resulting fibers or nonwoven fabric laminate, such as tensile strength, are lowered.

The propylene-based polymer for use in the MB nonwoven fabric 12 to constitute the nonwoven fabric laminate 10 has a melt flow rate (measured at a temperature of 230°C under a load of 2.16 kg, also referred to as "MFR" hereinafter) of preferably 200 to 3000 g/10 min, more preferably 500 to 2500 g/10 min. The reason is that if the MFR is less than the lower limit of the above range, there is a fear that spinning becomes difficult when fibers of small fineness are obtained, and if the MFR is more than the upper limit of the above range, there is a fear that fibers are not formed but small resin lumps called shots are formed in the spinning, and hence, shielding property is lowered because of deterioration of texture (uniformity) and a rough feeling of the surface of the resulting nonwoven fabric actually develops.

The propylene-based polymer fibers to constitute the first SB nonwoven fabric 14 and the second SB nonwoven fabric 16 have a fiber diameter of usually not more than 1.56 dtex (1.4 deniers), preferably 0.11 to 0.89 dtex (0.1 to 0.8 denier). If the fiber diameter of the propylene-based polymer fibers is in the above range, the resulting nonwoven fabric laminate is rich in softness and bleed-through of a hot melt can be also inhibited, so that such a fiber diameter is desirable. Provided that the fiber diameter is in the above range, the first SB nonwoven fabric and the second SB nonwovne fabric may be different from each other in fiber diameter.

The propylene-based polymer fibers to constitute the MB nonwoven fabric 12 have a fiber diameter of usually 1 $\mu$m to 5 $\mu$m, preferably 1 $\mu$m to 2 $\mu$m. By constituting the MB nonwoven fabric 12 from fine fibers having a fiber diameter of the above range, a nonwoven fabric layer of close texture that has excellent shielding property and is rich in softness is obtained. The MB nonwoven fabric 12 can be prepared by a hitherto publicly known process. For example, the MB nonwoven fabric 12 can be prepared by melt extruding the propylene-based polymer from a nozzle and applying hot air to form extra fine fibers. In order to make the fiber diameter of the MB nonwoven fabric smaller, it is necessary to decrease the discharge rate per nozzle hole, and this leads to lowering of productivity, so that the fiber diameter is preferably not less than 1 $\mu$m.

The nonwoven fabric laminate 10 of the invention has been partially compression bonded by hot embossing, and its compression bond area is in the range of 6 to 25%. If the area ratio exceeds the upper limit of the above range, 5% modulus strength of the nonwoven fabric laminate 10 is increased, and softness thereof tends to be deteriorated, so that such an area ratio is undesirable. If the area ratio is less than the lower limit of the above range, insufficiency of tensile strength and fluffing of the nonwoven fabric laminate 10 are apt to occur, so that such an area ratio is undesirable.

The overall basis weight of the nonwovne fabric laminate 10 of the invention is not more than 30 g/m$^2$. If the overall basis weight exceeds 30 g/m$^2$, the whole of the laminate becomes thick, and this tends to cause hard touch.

The nonwoven fabric laminate 10 of the invention has a 5% modulus index of usually 0.15 to 0.38 N/30 mm/(g/m$^2$). The nonwoven fabric laminate having such a 5% modulus index is rich in softness, has high strength and is stable. Therefore, it can be used as a moisture-permeable nonwoven fabric laminated sheet by laminating it onto a moisture-permeable film.

In the present invention, the basis weight of the MB nonwoven fabric 12 to constitute the nonwoven fabric laminate 10 is less than 3 g/m$^2$, preferably 0.05 to 0.9 g/m$^2$. If the basis weight exceeds the upper limit of the above range, hard texture of the MB nonwoven fabric 12 actually develops as texture of the whole of the nonwoven fabric laminate 10, so that such basis weight is undesirable.

The basis weight of the first SB nonwoven fabric 14 to constitute the nonwoven fabric laminate 10 of the invention is in the range of usually 3 to 25 g/m$^2$, and the basis weight of the second SB nonwoven fabric 16 is in the range of usually 1 to 11 g/m$^2$. In the nonwoven fabric laminate 10 of the invention, the ratio of the basis weight of the first SB nonwoven fabric 14 to the basis weight of the second SB nonwoven fabric 16 (basis weight of first SB nonwoven fabric/basis weight of second SB nonwoven fabric) (referred to as "basis weight ratio" hereinafter) is not less than 1.6, preferably 1.8 to 3.0, and the ratio of a thickness of the first SB nonwoven fabric 14 to a thickness of the second SB nonwoven fabric 16 (thickness of first SB nonwoven fabric/thickness of second SB nonwoven fabric) (referred to as "thickness ratio" hereinafter) is not less than 1.4, preferably 1.6 to 2.9. By allowing the first SB nonwoven fabric 14 to have larger basis weight and larger thickness than the second SB nonwoven fabric 16 according to the above conditions, it becomes possible to diffuse the hot melt adhesive into voids of the first SB nonwoven fabric 14 and thereby to decrease the quantity of the hot melt adhesive that reaches the MB nonwoven fabric 12 when the hot melt adhesive is applied to bond the nonwoven fabric laminate to a moisture-permeable film, and a small quantity of the hot melt adhesive that reaches the MB nonwoven fabric can be shielded by the MB nonwoven fabric 12 to completely block penetration of the hot melt adhesive.

So long as the basis weight, the basis weight ratio, the thickness ratio are in the above ranges, each or any one of the first SB nonwoven fabric and the second SB nonwoven fabric may be a layer wherein two or more SB nonwoven fabrics are laminated.

The moisture-permeable nonwoven fabric laminated sheet 20 of the invention has a structure wherein a moisture-permeable film 24 is bonded to the hot melt adhesive application predetermined surface 18 of the nonwoven fabric laminate 10 with a hot melt adhesive 22, as shown in Fig. 2.

As the hot melt adhesive 22, an olefin-based polymer (ethylene/propylene copolymer), a styrene block copolymer-based polymer (SEBS, SEPS, SIS, SBS), polyamide, polyester, a urethane-based polymer hot melt resin or the like is preferably used. These hot melt adhesives, however, are examples, and various hot melt adhesives publicly known are employable without limiting thereto.

Examples of the moisture-permeable films 24 include a film having moisture permeability in itself, a film imparted with moisture permeability by stretching a film and thereby forming fine pores, and a film obtained by stretching a film that is obtained by melt extrusion of a polyolefin resin containing a filler and thereby forming fine pores. Examples of such polyolefin resins include high-density polyethylene, low-density polyethylene, linear low-density polyethylene, an ethylene/$\alpha$-olefin copolymer, polypropylene, a propylene/$\alpha$-olefin copolymer, and polyolefin resins composed of mixtures thereof. The materials of the moisture-permeable films may be used singly or in combination of two or more kinds. The filler used herein is not specifically restricted, and any of inorganic fillers and organic fillers may be used. Examples of the inorganic fillers include oxides, hydroxides, carbonates, sulfates and silicates of alkaline earth metals and periodic table III group elements. Examples of the organic fillers include cellulose powders, such as wood flour and pulp, silicone and crosslinked substance powders, such as of phenol. These fillers may be used singly or in combination of two or more kinds. Of these, calcium carbonate, barium sulfate and the like are preferable, taking cost and stability of quality into account. The resin composition used for producing the moisture-permeable film may contain additives, such as antioxidant, ultraviolet light absorber, antibacterial agent, mildewproofing agent, rust proof agent, lubricant, pigment and heat stabilizer, within limits not detrimental to the object of the invention.

The moisture-permeable nonwoven fabric laminated sheet 20 has a structure wherein the hot melt adhesive application predetermined surface 18 and the moisture-permeable film 24 are bonded with the hot melt adhesive 22, and when they are bonded, the hot melt adhesive 22 is diffused into voids of the first SB nonwoven fabric 14 from the hot melt adhesive application predetermined surface 18 to decrease the quantity of the hot melt adhesive that reaches the MB nonwoven fabric 12. In the moisture-permeable nonwoven fabric laminated sheet 20, therefore, bleed-through of the adhesive less occurs than in the conventional laminated sheets.

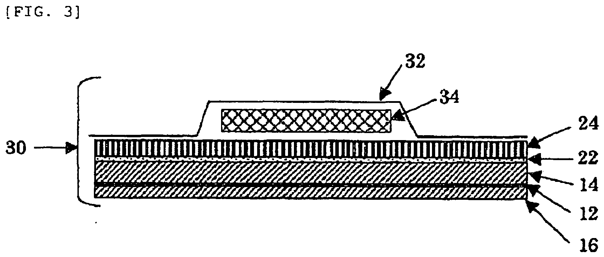

Examples of sanitary products using the moisture-permeable nonwoven fabric laminated sheet 20 as a backsheet include disposable diapers for babies or adults, disposable pants for children or adults, and sanitary napkins. In Fig. 3, a schematic side view of an absorbing part of a disposable diaper that is a typical example of the sanitary product of the invention is shown.

The moisture-permeable nonwoven fabric laminated sheet is used for an absorbing article in such a manner that an absorber 34 is interposed between a liquid-permeable top sheet 32 and a liquid-impermeable backsheet, as shown in Fig. 3. When the moisture-permeable nonwoven fabric laminated sheet is used for a diaper, the first SB nonwoven fabric 14 having larger basis weight and larger thickness than the second SB nonwonve fabric 16 is positioned closer to the skin than the second SB nonwoven fabric, as shown in Fig. 3, and therefore, this laminated sheet has a better feeling in use than a conventional laminated sheet having the same overall basis weight.

The present invention further includes a sanitary product comprising the nonwoven fabric laminate, a sanitary product comprising the moisture-permeable nonwoven fabric laminated sheet, and a sanitary product comprising the nonwoven fabric laminate and the nonwoven fabric laminated sheet.

Such a sanitary product is, for example, a sanitary product using the nonwoven fabric laminate or the nonwoven fabric laminated sheet as a solid gather, an absorber wrapping material, a top sheet, a liquid diffusion sheet, a selvedge member or the like.

The composite nonwoven fabric laminated sheet of the invention is obtained by laminating at least one sheet material selected from the group consisting of a nonwoven fabric, a woven fabric, a paper, a waterproof sheet, a net and a synthetic resin sheet on a surface of the first spunbonded nonwoven fabric of the nonwoven fabric laminate through a hot melt adhesive.

The sanitary product comprising the composite nonwoven fabric laminated sheet of the invention is, for example, a sanitary product comprising the composite nonwoven fabric laminated sheet as a solid gather

The composite nonwoven fabric laminated sheet can be used for, for example, medical gowns, masks, bed mats, wipers for life-related materials, filters for industrial materials, wipers, interior or exterior trim materials for automobile materials, sound absorbing materials, spring covers, agricultural direct-covering materials, greenhouse linings and fruit bags.

EXAMPLES

The present invention is further described with reference to the following examples, but it should be construed that the invention is in no way limited to those examples.

Properties are evaluated or measured in accordance with the following methods.

(1) Spunbonded fiber diameter (dtex, number of grams of fiber based on length of 10000 m (denier, d: based on length of 9000 m))

1 dtex = 1 g/fiber length 10000 m = 10/9 d (1 d = 1 g/fiber length 9000 m)

From the nonwoven fabric laminate sample prepared, 10 test specimens each having a size of 10 mm x 10 mm were picked. The picking places in the MD direction were arbitrarily selected, and those in the CD direction were 10 places present on a straight line at regular intervals, excluding both ends 20 cm of the nonwoven fabric laminate sample.
Using a Nikon ECLIPSE E400 microscope of 20 magnifications, the diameter of the spunbonded fiber was read out up to one decimal place in a unit of $\mu$m. Diameters at arbitrary 20 places were measured for each test specimen. These measurements were carried out on each of the 10 test specimens (diameter measuring points: 200 in all). From the results of diameter measurements, the number of grams of the fiber based on 9000 m at every measuring point was calculated. In this calculation, the density of polypropylene was set to 0.91 g/cm$^3$.
The number of grams of the fiber based on 9000 m at each of 200 measuring points was individually converted, then the mean value of the converted values was determined, and a value obtained by rounding the number of the mean value to one decimal place was regarded as a spunbonded fiber diameter of each nonwoven fabric sample.
The above measurement was carried out on both surfaces of the first SB nonwoven fabric 14 and the second SB nonwoven fabric 16.

(2) Melt blown fiber diameter

From the nonwoven fabric laminate sample prepared, 10 test specimens each having a size of 10 mm $\times$ 10 mm were picked. The picking places in the MD direction were arbitrarily selected, and those in the CD direction were 10 places present on a straight line at regular intervals, excluding both ends 20 cm of the nonwoven fabric laminate sample.
Using a Hitachi electron microscope S-3500N model of 15,000 magnifications, the diameter of the melt blown fiber was read out up to two decimal place in a unit of $\mu$m. Diameters at arbitrary 20 places were measured for each test specimen. These measurements were carried out on each of the 10 test specimens (diameter measuring points: 200 in all).
The mean value of the measured values at 200 measuring points was determined, and a value obtained by rounding the number of the mean value to the units digit was regarded as a melt blown fiber diameter of each nonwoven fabric laminate sample.

(3) 5% Modulus index

The nonwoven fabric laminate sample prepared was divided into 5 equal parts in the CD direction and divided into 3 equal parts in the MD direction, excluding both ends 20 cm of the nonwoven fabric laminate sample, and with regard to the resulting 15 parts, test specimens each having a size of 30 mm (CD direction) $\times$ 200 mm (MD direction) were sampled. The test specimen was set in a grasp length on a tensile tester of low-speed elongation test type, and a load was given at a pulling rate of 300 mm/min until the test specimen was broken. The mean value of strengths at the time of 5% elongation of the test specimens in each of the MD direction and the CD direction was determined (rounded to one decimal place). The resulting value was regarded as a 5% modulus, and a 5% modulus index was calculated from the following formula.

$$5\% \text{ Modulus index} = 5\% \text{ modulus [N/30 mm]/overall basis weight [g/m}^2\text{]}$$

(4) Air permeability

The nonwoven fabric laminate sample prepared was divided into 5 equal parts in the CD direction and divided into 3 equal parts in the MD direction, excluding both ends 20 cm of the nonwoven fabric laminate sample, and with regard to the resulting 15 parts, test specimens each having a size of 200 mm square were sampled. The test specimens were subjected to measurements in accordance with the air permeability test method A (Frazier method) of JIS L 1092-1999, 8.27.1, and from the mean value thereof, air permeability [cc/cm$^2$/s] was calculated.

(5) Water pressure resistance

The nonwoven fabric laminate sample prepared was divided into 5 equal parts in the CD direction and divided into 3 equal parts in the MD direction, excluding both ends 20 cm of the nonwoven fabric laminate sample, and with regard to the resulting 15 parts, test specimens each having a size of 200 mm square were sampled. The test specimens were subjected to measurements in accordance with the hydrostatic pressure method of the water resistance test method A of JIS L 1092-1992, 5.1, and from the mean value thereof, water pressure resistance [Pa]([MmAq]) was calculated.

(6) Softness

The nonwoven fabric laminate sample prepared was touched with a hand to examine a feeling, and the sample was evaluated by the following criteria.

AA: The nonwoven fabric laminate sample is extremely soft and has no crisp feeling.
BB: The nonwoven fabric laminate sample is soft and hardly has a crisp feeling.
CC: The nonwoven fabric laminate sample is hard and has a crisp feeling.

(7) Bleed-through of hot melt adhesive

The moisture-permeable nonwoven fabric laminated sheet having been wound into a roll was rewound, and with regard to the moisture-permeable nonwoven fabric laminated sheets superposed upon each other, the degree of adhesion between the front surface of the nonwoven fabric laminate of one moisture-permeable nonwoven fabric laminated sheet and the back surface of the nonwoven fabric laminate of the other moisture-permeable nonwoven fabric laminated sheet was visually observed, followed by evaluation by the following criteria.

AA: There is no bleed-through of a hot melt adhesive.
BB: There is slight bleed-through of a hot melt adhesive, but the bleed-through portion is free from film break (pinhole).
CC: There is bleed-through of a hot melt adhesive, and the bleed-through portion suffers film break (pinhole).

Example 1

As a spunbonded nonwoven fabric raw material, polypropylene (propylene homopolymer, MFR (measured at a temperature of 230°C under a load of 2.16 kg in accordance with JIS K7210-1999): 65 g/10 min) was used. Under the conditions of a molten resin temperature of 220°C, a single hole discharge rate of 0.40 g/min, a cooling air flow velocity of 1.4 m/s and a cooling air flow temperature of 25°C, two layers of spunbonded nonwoven fabrics having a fiber diameter of 1.33 dtex (1.2 d) were laminated upon each other to obtain a first SB nonwoven fabric 14 having basis weight of 10.8 g/m$^2$.

As a melt blown nonwoven fabric raw material, polypropylene (propylene homopolymer, MFR (measured at a temperature of 230°C under a load of 2.16 kg in accordance with JIS K7210-1999): 900 g/10 min) was used. The raw material was melt kneaded by an extruder at a molding temperature of 290°C, and the resulting melt kneadate was extruded into a high-speed hot air stream from a melt blowing die to obtain a MB nonwoven fabric having a fiber diameter of 2 $\mu$m and basis weight of 0.9 g/m$^2$.

A second SB nonwoven fabric 16 having a fiber diameter of 1.33 dtex (1.2 d) and basis weight of 5.3 g/m$^2$ was obtained in the same manner as for the first SB nonwoven fabric 14, except that the number of layers was changed to one.

The first SB nonwoven fabric 14, the MB nonwoven fabric 12 and the second SB nonwoven fabric 16 were in-line laminated in this order, and the resulting laminate was passed between an embossing roll (compression bond area ratio: 18%) and a flat roll to perform hot embossing with controlling a pressure and a temperature. Thus, a nonwoven fabric laminate 10 having a thickness of the first SB nonwoven fabric 14 of 163 $\mu$m, a thickness of the second SB nonwoven fabric 16 of 90 $\mu$m, a basis weight ratio of 2.0, a thickness ratio of 1.8 and overall basis weight of 17 g/m$^2$ was obtained. Then, a 5% modulus index, air permeability, water pressure resistance and softness of the nonwoven fabric laminate 10 were measured.

40 Parts by weight of linear low-density polyethylene (density: 0.924 g/cm$^2$, MFR: 2 g/10 min (190°C)) and 60 parts by weight of calcium carbonate (mean particle diameter: 1.7 $\mu$m) were mixed and stirred by a tumbler to prepare a resin composition. The resulting resin composition was fed to a twin-screw extruder, melt kneaded at a cylinder temperature of 230°C and then extruded from a T-die at a die temperature of 230°C to produce a film. The resulting film was monoaxially stretched under the conditions of a stretching temperature of 80°C, a stretching rate of 20 m/min and a stretch ratio of 3 times to obtain a moisture-permeable film 24 having a thickness of 25 $\mu$m.

The hot melt adhesive application predetermined surface 18 of the nonwoven fabric laminate 10 was coated with an olefin-based hot melt adhesive 22 in a coating weight of 3 g/m$^2$, and the moisture-permeable film 24 was laminated thereto to prepare a moisture-permeable nonwoven fabric laminated sheet 20. The moisture-permeable nonwoven fabric laminated sheet 20 was measured on bleed-through of the hot melt adhesive.

The results are set forth in Table 1.

Example 2

A first SB nonwoven fabric 14 having a fiber diameter of 1.56 dtex (1.4 d) and basis weight of 9.4 g/m$^2$ and a second SB nonwoven fabric 16 having a fiber diameter of 1.56 dtex (1.4 d) and basis weight of 4.7 g/m$^2$ were each obtained by controlling the molding conditions in Example 1.

Then, a MB nonwoven fabric 12 having a fiber diameter of 2 $\mu$m and basis weight of 0.9 g/m$^2$ was obtained.

By making other conditions the same as those in Example 1, a nonwoven fabric laminate 10 (thickness of first SB nonwoven fabric 14: 148 $\mu$m, thickness of second SB nonwoven fabric 16: 81 $\mu$m, basis weight ratio: 2.0, thickness ratio: 1.8, overall basis weight: 15 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.

The results are set forth in Table 1.

Examples 3 to 5

A first SB nonwoven fabric 14 having basis weight of 9.4 g/m$^2$ (Example 3), 8.7 g/m$^2$ (Example 4) or 10.6 g/m$^2$ (Example 5) and a second SB nonwoven fabric 16 having basis weight of 4.7 g/m$^2$ (Example 3), 5.4 g/m$^2$ (Example 4) or 3.5 g/m$^2$ (Example 5) were each obtained by controlling the molding conditions in Example 1.

Then, a MB nonwoven fabric 12 having a fiber diameter of 2 $\mu$m and basis weight of 0.9 g/m$^2$ was obtained.

By making other conditions the same as those in Example 1, a nonwoven fabric laminate 10 (thickness of first SB nonwoven fabric 14: 143 $\mu$m (Example 3), 130 $\mu$m (Example 4), 153 $\mu$m (Example 5); thickness of second SB nonwoven fabric 16: 78 $\mu$m (Example 3), 92 $\mu$m (Example 4), 69 $\mu$m (Example 5); basis weight ratio: 2.0 (Example 3), 1.6 (Example 4), 3.0 (Example 5); thickness ratio: 1.8 (Example 3), 1.4 (Example 4), 2.2 (Example 5); overall basis weight: 15 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.

The results are set forth in Table 1.

Examples 6 to 8

A first SB nonwoven fabric 14 having a fiber diameter of 0.89 dtex (0.8 d) and basis weight of 9.4 g/m$^2$ (Example 6), 10.6 g/m$^2$ (Example 7) or 8.7 g/m$^2$ (Example 8) and a second SB nonwoven fabric 16 having a fiber diameter of 0.89 dtex (0.8 d) and basis weight of 4.7 g/m$^2$ (Example 6), 3.5 g/m$^2$ (Example 7) or 5.4 g/m$^2$ (Example 8) were each obtained by controlling the molding conditions in Example 1.

Then, a MB nonwoven fabric 12 having a fiber diameter of 2 $\mu$m and basis weight of 0.9 g/m$^2$ was obtained.

By making other conditions the same as those in Example 1, a nonwoven fabric laminate 10 (thickness of first SB nonwoven fabric 14: 134 $\mu$m (Example 6), 140 $\mu$m (Example 7), 123 $\mu$m (Example 8); thickness of second SB nonwoven fabric 16: 74 $\mu$m (Example 6), 67 $\mu$m (Example 7), 89 $\mu$m (Example 8); basis weight ratio: 2.0 (Example 6), 3.0 (Example 7), 1.6 (Example 8); thickness ratio: 1.8 (Example 6), 2.1 (Example 7), 1.4 (Example 8); overall basis weight: 15 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.

The results are set forth in Table 1.

Example 9

A first SB nonwoven fabric 14 having a fiber diameter of 0.89 dtex (0.8 d) and basis weight of 8.7 g/m$^2$ and a second SB nonwoven fabric 16 having a fiber diameter of 0.89 dtex (0.8 d) and basis weight of 4.3 g/m$^2$ were each obtained by controlling the molding conditions in Example 1.

Then, a MB nonwoven fabric 12 having a fiber diameter of 2 $\mu$m and basis weight of 2.0 g/m$^2$ was obtained.

By making other conditions the same as those in Example 1, a nonwoven fabric laminate 10 (thickness of first SB nonwoven fabric 14: 125 $\mu$m, thickness of second SB nonwoven fabric 16: 68 $\mu$m, basis weight ratio of nonwoven fabric laminate 10: 2.0, thickness ratio: 1.8, overall basis weight: 15 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.
The results are set forth in Table 1.

Example 10

A first SB nonwoven fabric 14 having a fiber diameter of 0.56 dtex (0.5 d) and basis weight of 8.7 g/m$^2$ and a second SB nonwoven fabric 16 having a fiber diameter of 0.56 dtex (0.5 d) and basis weight of 5.4 g/m$^2$ were each obtained by controlling the molding conditions in Example 1.

Then, a MB nonwoven fabric 12 having a fiber diameter of 2 $\mu$m and basis weight of 0.9 g/m$^2$ was obtained.

By making other conditions the same as those in Example 1, a nonwoven fabric laminate 10 (thickness of first SB nonwoven fabric 14: 117 $\mu$m, thickness of second SB nonwoven fabric 16: 85 $\mu$m, basis weight ratio: 1.6, thickness ratio: 1.4, overall basis weight: 15 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.

The results are set forth in Table 1.

Examples 11 to 14

A first SB nonwoven fabric 14 having a fiber diameter of 1,33 dtex (1.2 d) and basis weight of 7.1 g/m$^2$ (Example 11), 8.1 g/m$^2$ (Example 12), 9.1 g/m$^2$ (Example 13) or 7.5 g/m$^2$ (Example 14) and a second SB nonwoven fabric 16 having a fiber diameter of 1,33 dtex (1.2 d) and basis weight of 3.9 g/m$^2$ (Example 11), 4.0 g/m$^2$ (Example 12), 3.0 g/m$^2$ (Example 13) or 4.6 g/m$^2$ (Example 14) were each obtained by controlling the molding conditions in Example 1.

Then, a MB nonwoven fabric 12 having a fiber diameter of 2 $\mu$m and basis weight of 2.0 g/m$^2$ (Example 11) or 0.9 g/m$^2$ (Examples 12 to 14) was obtained.

By making other conditions the same as those in Example 1, a nonwoven fabric laminate 10 (thickness of first SB nonwoven fabric 14: 109 $\mu$m (Example 11), 122 $\mu$m (Example 12), 133 $\mu$m (Example 13), 111 $\mu$m (Example 14); thickness of second SB nonwoven fabric 16: 67 $\mu$m (Example 11), 67 $\mu$m (Example 12), 53 $\mu$m (Example 13), 78 $\mu$m (Example 14); basis weight ratio of nonwoven fabric laminate 10: 1.8 (Example 11), 2.0 (Example 12), 3.0 (Example 13), 1.6 (Example 14); thickness ratio: 1.6 (Example 11), 1.8 (Example 12), 2.5 (Example 13), 1.4 (Example 14); overall basis weight: 13 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.

The results are set forth in Table 2.

Examples 15 and 16

A first SB nonwoven fabric 14 having a fiber diameter of 0.89 dtex (0.8 d) and basis weight of 8.1 g/m$^2$ (Example 15) or 7.5 g/m$^2$ (Example 16) and a second SB nonwoven fabric 16 having a fiber diameter of 0.89 dtex (0.8 d) and basis weight of 4.0 g/m$^2$ (Example 15) or 4.6 g/m$^2$ (Example 16) were each obtained by controlling the molding conditions in Example 1.

Then, a MB nonwoven fabric 12 having a fiber diameter of 2 $\mu$m and basis weight of 0.9 g/m$^2$ was obtained.

By making other conditions the same as those in Example 1, a nonwoven fabric laminate 10 (thickness of first SB nonwoven fabric 14: 115 $\mu$m (Example 15), 104 $\mu$m (Example 16); thickness of second SB nonwoven fabric 16: 63 $\mu$m (Example 15), 75 $\mu$m (Example 16); basis weight ratio of nonwoven fabric laminate 10: 2.0 (Example 15), 1.6 (Example 16); thickness ratio: 1.8 (Example 15), 1.4 (Example 16); overall basis weight: 13 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.

The results are set forth in Table 2.

Examples 17 and 18

A first SB nonwoven fabric 14 having a fiber diameter of 0.56 dtex (0.5 d) and basis weight of 8.1 g/m$^2$ (Example 17) or 7.5 g/m$^2$ (Example 18) and a second SB nonwoven fabric 16 having a fiber diameter of 0.56 dtex (0.5 d) and basis weight of 4.0 g/m$^2$ (Example 17) or 4.6 g/m$^2$ (Example 18) were each obtained by controlling the molding conditions in Example 1.

Then, a MB nonwoven fabric 12 having a fiber diameter of 2 $\mu$m and basis weight of 0.9 g/m$^2$ was obtained.

By making other conditions the same as those in Example 1, a nonwoven fabric laminate 10 (thickness of first SB nonwoven fabric 14: 110 μm (Example 17), 101 μm (Example 18);thickness of second SB nonwoven fabric 16: 62 μm (Example 17), 73 μm (Example 18); basis weight ratio of nonwoven fabric laminate 10: 2.0 (Example 17), 1.6 (Example 18); thickness ratio: 1.8 (Example 17), 1.4 (Example 18); overall basis weight: 13 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.

The results are set forth in Table 2.

Example 19

A first SB nonwoven fabric 14 having a fiber diameter of 1.33 dtex (1.2 d) and basis weight of 6.1 g/m$^2$ and a second SB nonwoven fabric 16 having a fiber diameter of 1.33 dtex (1.2 d) and basis weight of 3.0 g/m$^2$ were each obtained by controlling the molding conditions in Example 1.

Then, a MB nonwoven fabric 12 having a fiber diameter of 2 μm and basis weight of 0.9 g/m$^2$ was obtained.

By making other conditions the same as those in Example 1, a nonwoven fabric laminate 10 (thickness of first SB nonwoven fabric 14: 90 μm, thickness of second SB nonwoven fabric 16: 49 μm, basis weight ratio of nonwoven fabric laminate 10: 2.0, thickness ratio: 1.8, overall basis weight: 10 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.

The results are set forth in Table 2.

Examples 20 to 22

A first SB nonwoven fabric 14 having a fiber diameter of 0.89 dtex (0.8d) and basis weight of 6.1 g/m$^2$ (Example 20), 6.9 g/m$^2$ (Example 21) or 5.6 g/m$^2$ (Example 22) and a second SB nonwoven fabric 16 having a fiber diameter of 0.89 dtex (0.8 d) and basis weight of 3.0 g/m$^2$ (Example 20), 2.3 g/m$^2$ (Example 21) or 3.5 g/m$^2$ (Example 22) were each obtained by controlling the molding conditions in Example 1.

Then, a MB nonwoven fabric 12 having a fiber diameter of 2 μm and basis weight of 0.9 g/m$^2$ was obtained.

By making other conditions the same as those in Example 1, a nonwoven fabric laminate 10 (thickness of first SB nonwoven fabric 14: 87 μm (Example 20), 97 μm (Example 21), 80 μm (Example 22); thickness of second SB nonwoven fabric 16: 48 μm (Example 20), 37 μm (Example 21), 56 μm (Example 22); basis weight ratio of nonwoven fabric laminate 10: 2.0 (Example 20), 3.0 (Example 21), 1.6 (Example 22); thickness ratio: 1.8 (Example 20), 2.6 (Example 21), 1.4 (Example 22); overall basis weight: 10 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.

The results are set forth in Table 2 (Example 20) and Table 3 (Examples 21 and 22).

Examples 23 to 25

A first SB nonwoven fabric 14 having a fiber diameter of 0.56 dtex (0.5 d) and basis weight of 6.1 g/m$^2$ (Example 23), 6.9 g/m$^2$ (Example 24) or 6.9 g/m$^2$ (Example 25) and a second SB nonwoven fabric 16 having a fiber diameter of 0.56 dtex (0.5 d) and basis weight of 3.0 g/m$^2$ (Example 23), 2.3 g/m$^2$ (Example 24) or 2.3 g/m$^2$ (Example 25) were each obtained by controlling the molding conditions in Example 1.

Then, a MB nonwoven fabric 12 having basis weight of 0.9 g/m$^2$ and a fiber diameter of 2 μm (Examples 23 and 24) or 1 μm (Example 25) was obtained.

By making other conditions the same as those in Example 1, a nonwoven fabric laminate 10 (thickness of first SB nonwoven fabric 14: 85 μm (Example 23), 94 μm (Example 24), 94 μm (Example 25); thickness of second SB nonwoven fabric 16: 45 μm (Example 23), 38 μm (Example 24), 38 μm (Example 25); basis weight ratio of nonwoven fabric laminate 10: 2.0 (Example 23), 3.0 (Example 24), 3.0 (Example 25); thickness ratio: 1.9 (Example 23), 2.5 (Example 24), 2.5 (Example 25); overall basis weight: 10 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.

The results are set forth in Table 3.

Example 26

A first SB nonwoven fabric 14 having a fiber diameter of 0.89 dtex (0.8 d) and basis weight of 4.0 g/m$^2$ and a

second SB nonwoven fabric 16 having a fiber diameter of 0.89 dtex (0.8 d) and basis weight of 2.1 g/m$^2$ were each obtained by controlling the molding conditions in Example 1.

Then, a MB nonwoven fabric 12 having basis weight of 0.9 g/m$^2$ and a fiber diameter of 1 μm was obtained.

By making other conditions the same as those in Example 1, a nonwoven fabric laminate 10 (thickness of first SB nonwoven fabric 14: 58 μm, thickness of second SB nonwoven fabric 16: 34 μm, basis weight ratio of nonwoven fabric laminate 10: 1.9, thickness ratio: 1.7, overall basis weight: 7 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.

The results are set forth in Table 3.

## Example 27

A first SB nonwoven fabric 14 having a fiber diameter of 0.56 dtex (0.5 d) and basis weight of 4.1 g/m$^2$ and a second SB nonwoven fabric 16 having a fiber diameter of 0.56 dtex (0.5 d) and basis weight of 2.0 g/m$^2$ were each obtained by controlling the molding conditions in Example 1.

Then, a MB nonwoven fabric 12 having a fiber diameter of 1 μm and basis weight of 0.9 g/m$^2$ was obtained.

By making other conditions the same as those in Example 1, a nonwoven fabric laminate 10 (thickness of first SB nonwoven fabric 14: 63 μm, thickness of second SB nonwoven fabric 16: 33 μm, basis weight ratio of nonwoven fabric laminate 10: 2.1, thickness ratio: 1.9, overall basis weight: 7 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.

The results are set forth in Table 3.

## Example 28

A first SB nonwoven fabric 14 having a fiber diameter of 0.89 dtex (0.8 d) and basis weight of 14.3 g/m$^2$ and a second SB nonwoven fabric 16 having a fiber diameter of 0.89 dtex (0.8 d) and basis weight of 4.8 g/m$^2$ were each obtained by controlling the molding conditions in Example 1.

Then, a MB nonwoven fabric 12 having a fiber diameter of 1 μm and basis weight of 0.1 g/m$^2$ was obtained.

By making other conditions the same as those in Example 1, a nonwoven fabric laminate 10 (thickness of first SB nonwoven fabric 14: 220 μm, thickness of second SB nonwoven fabric 16: 76 μm, basis weight ratio of nonwoven fabric laminate 10: 3.0, thickness ratio: 2.9, overall basis weight: 19 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.

The results are set forth in Table 3.

## Example 29

A first SB nonwoven fabric 14 having a fiber diameter of 0.89 dtex (0.8 d) and basis weight of 11.1 g/m$^2$ and a second SB nonwoven fabric 16 having a fiber diameter of 0.89 dtex (0.8 d) and basis weight of 5.5 g/m$^2$ were each obtained by controlling the molding conditions in Example 1.

Then, a MB nonwoven fabric 12 having a fiber diameter of 1 μm and basis weight of 0.4 g/m$^2$ was obtained.

By making other conditions the same as those in Example 1, a nonwoven fabric laminate 10 (thickness of first SB nonwoven fabric 14: 168 μm, thickness of second SB nonwoven fabric 16: 86 μm, basis weight ratio of nonwoven fabric laminate 10: 2.0, thickness ratio: 1.8, overall basis weight: 17 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.

The results are set forth in Table 3.

## Examples 30 and 31

A first SB nonwoven fabric 14 having a fiber diameter of 0.89 dtex (0.8 d) and basis weight of 8.1 g/m$^2$ and a second SB nonwoven fabric 16 having a fiber diameter of 0.89 dtex (0.8 d) and basis weight of 4.0 g/m$^2$ were each obtained by controlling the molding conditions in Example 1.

Then, a MB nonwoven fabric 12 having a fiber diameter of 2 μm and basis weight of 0.9 g/m$^2$ was obtained.

A nonwoven fabric laminate 10 (thickness of first SB nonwoven fabric 14: 115 μm (Example 30), 115 μm (Example 31); thickness of second SB nonwoven fabric 16: 63 μm (Example 30), 63 μm (Example 31); basis weight ratio of nonwoven fabric laminate 10: 2.0 (Example 30), 2.0 (Example 31); thickness ratio: 1.8 (Example 30), 1.8 (Example 31); overall basis weight: 13 g/m$^2$) and a moisture-permeable nonwoven fabric laminated sheet 20 were prepared in the same manner as in Example 1, except that an embossing roll having an embossing pattern area ratio of 6% (Example 30) or 25% (Example 31). Then, properties of the nonwoven fabric laminate and the moisture-permeable nonwoven fabric laminated sheet were measured and evaluated in the same manner as in Example 1.

The results are set forth in Table 3.

Comparative Example 1

A first SB nonwoven fabric 14 having a fiber diameter of 4.4dtex(4.0d) and basis weight of 3.1 g/m$^2$ and a second SB nonwoven fabric 16 having a fiber diameter of 4.4dtex(4.0d) and basis weight of 3.0 g/m$^2$ were each obtained by controlling the molding conditions and changing the number of SB nonwoven fabric layers for constituting the first nonwoven fabric to one in Example 1.

Then, a MB nonwoven fabric 12 having a fiber diameter of 6 $\mu$m and basis weight of 0.9 g/m$^2$ was obtained.

By making other conditions the same as those in Example 1, a nonwoven fabric laminate 10 (thickness of first SB nonwoven fabric 14: 54 $\mu$m, thickness of second SB nonwoven fabric 16: 53 $\mu$m, basis weight ratio: 1.0, thickness ratio: 1.0, overall basis weight: 7 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.

The results are set forth in Table 4.

Comparative Example 2

A first SB nonwoven fabric 14 having a fiber diameter of 1.33 dtex (1.2 d) and basis weight of 8.7 g/m$^2$ and a second SB nonwoven fabric 16 having a fiber diameter of 1.33 dtex (1.2 d) and basis weight of 5.4 g/m$^2$ were each obtained in the same manner as in Example 1.

Then, a MB nonwoven fabric 12 having a fiber diameter of 2 $\mu$m and basis weight of 0.9 g/m$^2$ was obtained.

By performing thermal compression bonding using an embossing roll having a compression bond area ratio of 30%, a nonwoven fabric laminate 10 (thickness of first SB nonwoven fabric 14: 130 $\mu$m, thickness of second SB nonwoven fabric 16: 92 $\mu$m, basis weight ratio: 1.6, thickness ratio: 1.4, overall basis weight: 15 g/m$^2$) and a moisture-permeable nonwoven fabric laminated sheet 20 were prepared. Then, properties of the nonwoven fabric laminate and the moisture-permeable nonwoven fabric laminated sheet were measured and evaluated in the same manner as in Example 1.

The results are set forth in Table 4.

Table 1

| | | | Ex. 1 | Ex. 2 | Ex. 3 | Ex. 4 | Ex. 5 |
|---|---|---|---|---|---|---|---|
| First SB nonwoven fabric | Resin temperature | [°C] | 220 | 210 | 220 | 220 | 220 |
| | Single hole discharge rate | [g/min/hole] | 0.40 | 0.40 | 0.40 | 0.40 | 0.40 |
| | Cooling air flow velocity | [m/s] | 1.4 | 1.1 | 1.3 | 1.3 | 1.3 |
| | Fiber diameter | [dtex]([d]) | 1.33(1.2) | 1.56(1.4) | 1.33(1.2) | 1.33(1.2) | 1.33(1.2) |
| | Basis weight | [g/m$^2$] | 10.8 | 9.4 | 9.4 | 8.7 | 10.6 |
| | Thickness | [μm] | 163 | 148 | 143 | 130 | 153 |
| MB nonwoven fabric | Basis weight | [g/m$^2$] | 0.9 | 0.9 | 0.9 | 0.9 | 0.9 |
| | Fiber diameter | [μm] | 2 | 2 | 2 | 2 | 2 |
| Second SB nonwoven fabric | Resin temperature | [°C] | 220 | 210 | 220 | 210 | 230 |
| | Single hole discharge rate | [g/min/hole] | 0.40 | 0.40 | 0.40 | 0.50 | 0.27 |
| | Cooling air flow velocity | [m/s] | 1.4 | 1.1 | 1.3 | 1.7 | 0.9 |
| | Fiber diameter | [dtex]([d]) | 1.33(1.2) | 1.56(1.4) | 1.33(1.2) | 1.33(1.2) | 1.33(1.2) |
| | Basis weight | [g/m$^2$] | 5.3 | 4.7 | 4.7 | 5.4 | 3.5 |
| | Thickness | [μm] | 90 | 81 | 78 | 92 | 69 |
| Nonwoven fabric laminate | Overall basis weight | [g/m$^2$] | 17 | 15 | 15 | 15 | 15 |
| | Basis weight ratio (first SB/second SB) | [-] | 2.0 | 2.0 | 2.0 | 1.6 | 3.0 |
| | Thickness ratio (first SB/second SB) | [-] | 1.8 | 1.8 | 1.8 | 1.4 | 2.2 |
| | Compression bond area ratio | [%] | 18 | 18 | 18 | 18 | 18 |
| Properties | 5% Modulus index | [N/30mm/(g/m$^2$)] | 0.27 | 0.29 | 0.32 | 0.31 | 0.32 |
| | Air permeability | [cc/cm$^2$/s] | 184 | 190 | 182 | 184 | 184 |
| | Water pressure resistance | Pa ([mmAq]) | 1815 (185) | 1697 (173) | 1746 (178) | 1727 (176) | 1756 (179) |
| | Softness | [-] | BB | BB | BB | BB | BB |
| | Bleed-through of hot melt adhesive | [-] | AA | AA | AA | BB | AA |

Table 1 (Continued)

| | | | Ex. 6 | Ex. 7 | Ex. 8 | Ex. 9 | Ex. 10 |
|---|---|---|---|---|---|---|---|
| First SB nonwoven fabric | Resin temperature | [°C] | 235 | 235 | 235 | 235 | 245 |
| | Single hole discharge rate | [g/min/hole] | 0.32 | 0.32 | 0.32 | 0.32 | 0.22 |
| | Cooling air flow velocity | [m/s] | 1.6 | 1.6 | 1.6 | 1.6 | 1.8 |
| | Fiber diameter | [dtex]([d]) | 0.89 (0.8) | 0.89 (0.8) | 0.89 (0.8) | 0.89 (0.8 | 0.56 (0.5) |
| | Basis weight | [g/m$^2$] | 9.4 | 10.6 | 8.7 | 8.7 | 8.7 |
| | Thickness | [μm] | 134 | 140 | 123 | 125 | 117 |
| MB nonwoven fabric | Basis weight | [g/m$^2$] | 0.9 | 0.9 | 0.9 | 2.0 | 0.9 |
| | Fiber diameter | [μm] | 2 | 2 | 2 | 2 | 2 |
| Second SB nonwoven fabric | Resin temperature | [°C] | 235 | 240 | 230 | 235 | 240 |
| | Single hole discharge rate | [g/min/hole] | 0.32 | 0.21 | 0.40 | 0.32 | 0.28 |
| | Cooling air flow velocity | [m/s] | 1.6 | 1.1 | 2.0 | 1.6 | 2.2 |
| | Fiber diameter | [dtex]([d]) | 0.89 (0.8) | 0.89 (0.8) | 0.89 (0.8) | 0.89 (0.8 | 0.56 (0.5) |
| | Basis weight | [g/m$^2$] | 4.7 | 3.5 | 5.4 | 4.3 | 5.4 |
| | Thickness | [μm] | 74 | 67 | 89 | 68 | 85 |
| Nonwoven fabric laminate | Overall basis weight | [g/m$^2$] | 15 | 15 | 15 | 15 | 15 |
| | Basis weight ratio (first SB/second SB) | [-] | 2.0 | 3.0 | 1.6 | 2.0 | 1.6 |
| | Thickness ratio (first SB/second SB) | [-] | 1.8 | 2.1 | 1.4 | 1.8 | 1.4 |
| | Compression bond area ratio | [%] | 18 | 18 | 18 | 18 | 18 |
| Properties | 5% Modulus index | [N/30mm/(g/m$^2$)] | 0.33 | 0.33 | 0.34 | 0.15 | 0.35 |
| | Air permeability | [cc/cm$^2$/s] | 168 | 166 | 165 | 165 | 151 |
| | Water pressure resistance | Pa ([mmAq]) | 2021 (206) | 2031 (207) | 2040 (208) | 2040 (208) | 2197 (224) |
| | Softness | [-] | AA | AA | AA | BB | AA |
| | Bleed-through of hot melt adhesive | [-] | AA | AA | BB | AA | BB |

EP 1 980 390 B2

Table 2

| | | | Ex. 11 | Ex. 12 | Ex. 13 | Ex. 14 | Ex. 15 |
|---|---|---|---|---|---|---|---|
| First SB nonwoven fabric | Resin temperature | [°C] | 220 | 220 | 220 | 220 | 235 |
| | Single hole discharge rate | [g/min/hole] | 0.40 | 0.40 | 0.40 | 0.40 | 0.32 |
| | Cooling air flow velocity | [m/s] | 1.4 | 1.4 | 1.4 | 1.4 | 1.6 |
| | Fiber diameter | [dtex]([d]) | 1.33(1.2) | 1.33(1.2) | 1.33 (1.2) | 1.33(1.2) | 0.89 (0.8) |
| | Basis weight | [g/m$^2$] | 7.1 | 8.1 | 9.1 | 7.5 | 8.1 |
| | Thickness | [μm] | 109 | 122 | 133 | 111 | 115 |
| MB nonwoven fabric | Basis weight | [g/m$^2$] | 2.0 | 0.9 | 0.9 | 0.9 | 0.9 |
| | Fiber diameter | [μm] | 2 | 2 | 2 | 2 | 2 |
| Second SB nonwoven fabric | Resin temperature | [°C] | 220 | 220 | 230 | 210 | 235 |
| | Single hole discharge rate | [g/min/hole] | 0.44 | 0.40 | 0.27 | 0.50 | 0.32 |
| | Cooling air flow velocity | [m/s] | 1.5 | 1.4 | 0.9 | 1.7 | 1.6 |
| | Fiber diameter | [dtex]([d]) | 1.33(1.2) | 1.33(1.2) | 1.33 (1.2) | 1.33(1.2) | 0.89 (0.8) |
| | Basis weight | [g/m$^2$] | 3.9 | 4.0 | 3.0 | 4.6 | 4.0 |
| | Thickness | [μm] | 67 | 67 | 53 | 78 | 63 |
| Nonwoven fabric laminate | Overall basis weight | [g/m$^2$] | 13 | 13 | 13 | 13 | 13 |
| | Basis weight ratio (first SB/second SB) | [-] | 1.8 | 2.0 | 3.0 | 1.6 | 2.0 |
| | Thickness ratio (first SB/second SB) | [-] | 1.6 | 1.8 | 2.5 | 1.4 | 1.8 |
| | Compression bond area ratio | [%] | 18 | 18 | 18 | 18 | 18 |
| Properties | 5% Modulus index | [N/30mm/(g/m$^2$)] | 0.22 | 0.32 | 0.33 | 0.32 | 0.33 |
| | Air permeability | [cc/cm$^2$/s] | 161 | 173 | 175 | 175 | 170 |
| | Water pressure resistance | Pa ([mmAq]) | 1785 (182) | 1540 (157) | 1550 (158) | 1550 (158) | 1687 (172) |
| | Softness | [-] | BB | BB | BB | BB | AA |
| | Bleed-through of hot melt adhesive | [-] | AA | AA | AA | BB | AA |

Table 2 (Continued)

| | | | Ex. 16 | Ex. 17 | Ex. 18 | Ex. 19 | Ex. 20 |
|---|---|---|---|---|---|---|---|
| First SB nonwoven fabric | Resin temperature | [°C] | 235 | 245 | 245 | 220 | 235 |
| | Single hole discharge rate | [g/min/hole] | 0.32 | 0.22 | 0.22 | 0.40 | 0.32 |
| | Cooling air flow velocity | [m/s] | 1.6 | 1.8 | 1.8 | 1.4 | 1.6 |
| | Fiber diameter | [dtex]([d]) | 0.89 (0.8) | 0.56 (0.5) | 0.56 (0.5) | 1.33(1.2) | 0.89 (0.8) |
| | Basis weight | [g/m²] | 7.5 | 8.1 | 7.5 | 6.1 | 6.1 |
| | Thickness | [μm] | 104 | 110 | 101 | 90 | 87 |
| MB nonwoven fabric | Basis weight | [g/m²] | 0.9 | 0.9 | 0.9 | 0.9 | 0.9 |
| | Fiber diameter | [μm] | 2 | 2 | 2 | 2 | 2 |
| Second SB nonwoven fabric | Resin temperature | [°C] | 230 | 245 | 240 | 220 | 235 |
| | Single hole discharge rate | [g/min/hole] | 0.40 | 0.22 | 0.28 | 0.40 | 0.32 |
| | Cooling air flow velocity | [m/s] | 2.0 | 1.8 | 2.2 | 1.4 | 1.6 |
| | Fiber diameter | [dtex]([d]) | 0.89 (0.8) | 0.56 (0.5) | 0.56 (0.5) | 1.33(1.2) | 0.89 (0.8) |
| | Basis weight | [g/m²] | 4.6 | 4.0 | 4.6 | 3.0 | 3.0 |
| | Thickness | [μm] | 75 | 62 | 73 | 49 | 48 |
| Nonwoven fabric laminate | Overall basis weight | [g/m²] | 13 | 13 | 13 | 10 | 10 |
| | Basis weight ratio (first SB/second SB) | [-] | 1.6 | 2.0 | 1.6 | 2.0 | 2.0 |
| | Thickness ratio (first SB/second SB) | [-] | 1.4 | 1.8 | 1.4 | 1.8 | 1.8 |
| | Compression bond area ratio | [%] | 18 | 18 | 18 | 18 | 18 |
| Properties | 5% Modulus index | [N/30mm/(g/m²)] | 0.33 | 0.34 | 0.35 | 0.31 | 0.33 |
| | Air permeability | [cc/cm²/s] | 168 | 142 | 143 | 220 | 198 |
| | Water pressure resistance | Pa ([mmAq]) | 1697 (173) | 2001 (204) | 2001 (204) | 1187 (121) | 1393 (142) |
| | Softness | [-] | AA | AA | AA | BB | AA |
| | Bleed-through of hot melt adhesive | [-] | BB | AA | BB | AA | AA |

EP 1 980 390 B2

Table 3

| | | | Ex. 21 | Ex. 22 | Ex. 23 | Ex. 24 | Ex. 25 |
|---|---|---|---|---|---|---|---|
| First SB nonwoven fabric | Resin temperature | [°C] | 235 | 235 | 240 | 240 | 240 |
| | Single hole discharge rate | [g/min/hole] | 0.32 | 0.32 | 0.30 | 0.30 | 0.30 |
| | Cooling air flow velocity | [m/s] | 1.6 | 1.6 | 2.4 | 2.4 | 2.4 |
| | Fiber diameter | [dtex]([d]) | 0.89 (0.8) | 0.89 (0.8) | 0.56 (0.5) | 0.56 (0.5) | 0.56 (0.5) |
| | Basis weight | [g/m²] | 6.9 | 5.6 | 6.1 | 6.9 | 6.9 |
| | Thickness | [μm] | 97 | 80 | 85 | 94 | 94 |
| MB nonwoven fabric | Basis weight | [g/m²] | 0.9 | 0.9 | 0.9 | 0.9 | 0.9 |
| | Fiber diameter | [μm] | 2 | 2 | 2 | 2 | 1 |
| Second SB nonwoven fabric | Resin temperature | [°C] | 240 | 230 | 240 | 245 | 245 |
| | Single hole discharge rate | [g/min/hole] | 0.21 | 0.40 | 0.30 | 0.20 | 0.20 |
| | Cooling air flow velocity | [m/s] | 1.1 | 2.0 | 2.4 | 1.6 | 1.6 |
| | Fiber diameter | [dtex]([d]) | 0.89 (0.8) | 0.89 (0.8) | 0.56 (0.5) | 0.56 (0.5) | 0.56 (0.5) |
| | Basis weight | [g/m²] | 2.3 | 3.5 | 3.0 | 2.3 | 2.3 |
| | Thickness | [μm] | 37 | 56 | 45 | 38 | 38 |
| Nonwoven fabric laminate | Overall basis weight | [g/m²] | 10 | 10 | 10 | 10 | 10 |
| | Basis weight ratio (first SB/second SB) | [-] | 3.0 | 1.6 | 2.0 | 3.0 | 3.0 |
| | Thickness ratio (first SB/second SB) | [-] | 2.6 | 1.4 | 1.9 | 2.5 | 2.5 |
| | Compression bond area ratio | [%] | 18 | 18 | 18 | 18 | 18 |
| Properties | 5% Modulus index | [N/30mm/(g/m²)] | 0.34 | 0.33 | 0.34 | 0.35 | 0.34 |
| | Air permeability | [cc/cm²/s] | 198 | 198 | 173 | 176 | 160 |
| | Water pressure resistance | Pa ([mmAq]) | 1393 (142) | 1393 (142) | 1570 (160) | 1560 (159) | 1697 (173) |
| | Softness | [-] | AA | AA | AA | AA | AA |
| | Bleed-through of hot melt adhesive | [-] | AA | BB | AA | AA | AA |

EP 1 980 390 B2

Table 3 (Continued)

| | | | Ex. 26 | Ex. 27 | Ex. 28 | Ex. 29 | Ex. 30 | Ex. 31 |
|---|---|---|---|---|---|---|---|---|
| First SB nonwoven fabric | Resin temperature | [°C] | 230 | 245 | 230 | 230 | 235 | 235 |
| | Single hole discharge rate | [g/min/hole] | 0.32 | 0.22 | 0.32 | 0.32 | 0.32 | 0.32 |
| | Cooling air flow velocity | [m/s] | 1.8 | 1.8 | 1.8 | 1.8 | 1.6 | 1.6 |
| | Fiber diameter | [dtex]([d]) | 0.89 (0.8) | 0.56 (0.5) | 0.89 (0.8) | 0.89 (0.8) | 0.89 (0.8) | 0.89 (0.8) |
| | Basis weight | [g/m$^2$] | 4.0 | 4.1 | 14.3 | 11.1 | 8.1 | 8.1 |
| | Thickness | [μm] | 58 | 63 | 220 | 168 | 115 | 115 |
| MB nonwoven fabric | Basis weight | [g/m$^2$] | 0.9 | 0.9 | 0.1 | 0.4 | 0.9 | 0.9 |
| | Fiber diameter | [μm] | 1 | 1 | 1 | 1 | 2 | 2 |
| Second SB nonwoven fabric | Resin temperature | [°C] | 230 | 245 | 230 | 230 | 235 | 235 |
| | Single hole discharge rate | [g/min/hole] | 0.32 | 0.22 | 0.32 | 0.32 | 0.32 | 0.32 |
| | Cooling air flow velocity | [m/s] | 1.8 | 1.8 | 1.8 | 1.8 | 1.6 | 1.6 |
| | Fiber diameter | [dtex]([d]) | 0.89 (0.8) | 0.56 (0.5) | 0.89 (0.8) | 0.89 (0.8) | 0.89 (0.8) | 0.89 (0.8) |
| | Basis weight | [g/m$^2$] | 2.1 | 2.0 | 4.8 | 5.5 | 4.0 | 4.0 |
| | Thickness | [μm] | 34 | 33 | 76 | 86 | 63 | 63 |
| Nonwoven fabric laminate | Overall basis weight | [g/m$^2$] | 7 | 7 | 19 | 17 | 13 | 13 |
| | Basis weight ratio (first SB/second SB) | [-] | 1.9 | 2.1 | 3.0 | 2.0 | 2.0 | 2.0 |
| | Thickness ratio (first SB/second SB) | [-] | 1.7 | 1.9 | 2.9 | 1.8 | 1.8 | 1.8 |
| | Compression bond area ratio | [%] | 18 | 18 | 18 | 18 | 6 | 25 |
| Properties | 5% Modulus index | [N/30mm/(g/m$^2$)] | 0.33 | 0.34 | 0.28 | 0.29 | 0.15 | 0.38 |
| | Air permeability | [cc/cm$^2$/s] | 196 | 182 | 224 | 206 | 172 | 167 |
| | Water pressure resistance | Pa ([mmAq]) | 1403 (143) | 1501 (153) | 1334 (136) | 1403 (143) | 1658 (169) | 1678 (171) |
| | Softness | [-] | AA | AA | AA | AA | AA | AA |
| | Bleed-through of hot melt adhesive | [-] | AA | AA | AA | AA | AA | AA |

EP 1 980 390 B2

Table 4

| | | | Comp. Ex. 1 | Comp. Ex. 2 |
|---|---|---|---|---|
| First SB nonwoven fabric | Resin temperature | [°C] | 200 | 220 |
| | Single hole discharge rate | [g/min/hole] | 0.40 | 0.40 |
| | Cooling air flow velocity | [m/s] | 0.7 | 1.3 |
| | Fiber diameter | [dtex]([d]) | 4.44 (4.0) | 1.33 (1.2) |
| | Basis weight | [g/m$^2$] | 3.1 | 8.7 |
| | Thickness | [μm] | 54 | 130 |
| MB nonwoven fabric | Basis weight | [g/m$^2$] | 0.9 | 0.9 |
| | Fiber diameter | [μm] | 6 | 2 |
| Second SB nonwoven fabric | Resin temperature | [°C] | 200 | 210 |
| | Single hole discharge rate | [g/min/hole] | 0.40 | 0.50 |
| | Cooling air flow velocity | [m/s] | 0.7 | 1.7 |
| | Fiber diameter | [dtex]([d]) | 4.44 (4.0) | 1.33 (1.2) |
| | Basis weight | [g/m$^2$] | 3.0 | 5.4 |
| | Thickness | [μm] | 53 | 92 |
| Nonwoven fabric laminate | Overall basis weight | [g/m$^2$] | 7 | 15 |
| | Basis weight ratio (first SB/second SB) | [-] | 1.0 | 1.6 |
| | Thickness ratio (first SB/second SB) | [-] | 1.0 | 1.4 |
| | Compression bond area ratio | [%] | 18 | 30 |
| Properties | 5% Modulus index | [N/30mm/ (g/m$^2$)] | 0.10 | 0.41 |
| | Air permeability | [cc/cm$^2$/s] | 480 | 183 |
| | Water pressure resistance | Pa ([mmAq]) | 314 (32) | 1717 (175) |
| | Softness | [-] | CC | CC |
| | Bleed-through of hot melt adhesive | [-] | CC | BB |

**Claims**

1. A nonwoven fabric laminate comprising:

   a first spunbonded nonwoven fabric comprising a propylene-based polymer,
   a melt blown nonwoven fabric comprising a propylene-based polymer and laminated on the first spunbonded nonwoven fabric, and
   a second spunbonded nonwoven fabric comprising a propylene-based polymer and laminated on the melt blown nonwoven fabric,
   wherein the first spun-bonded nonwoven fabric, the melt-blown nonwoven fabric and the second spun-bonded nonwoven fabric are laminated in this order;
   the nonwoven fabric laminate having overall basis weight of not more than 30 g/m$^2$,
   wherein the basis weight of the first spunbonded nonwoven fabric is in the range of 3 to 25 g/m$^2$, the basis weight of the second spunbonded nonwoven fabric is in the range of 1 to 11 g/m$^2$, the ratio of the basis weight of the first spunbonded nonwoven fabric to the basis weight of the second spunbonded nonwoven fabric (basis weight of first spunbonded nonwoven fabric/basis weight of second spunbonded nonwoven fabric) is not less than 1.6, the ratio of a thickness of the first spunbonded nonwoven fabric to a thickness of the second spunbonded nonwoven fabric (thickness of first spunbonded nonwoven fabric/thickness of second spunbonded nonwoven fabric) is not less than 1.4, the basis weight of the melt blown nonwoven fabric is less than 3 g/m$^2$, and the

compression bond area ratio is in the range of 6 to 25%.

2. The nonwoven fabric laminate as claimed in claim 1, wherein fibers to form the first spunbonded nonwoven fabric and the second spunbonded nonwoven fabric have a fiber diameter of not more than 1.56 dtex (1.4 deniers), and fibers to form the melt blown nonwoven fabric have a fiber diameter of 1 to 5 $\mu$m.

3. The nonwoven fabric laminate as claimed in claim 1 or 2, which has a 5% modulus index of 0.15 to 0.38 N/30 mm/(g/m$^2$).

4. A composite nonwoven fabric laminated sheet obtained by laminating at least one sheet material selected from the group consisting of a nonwoven fabric, a woven fabric, a paper, a waterproof sheet, a net and a synthetic resin sheet or a moisture-permeable film on a surface of the first spunbonded nonwoven fabric of the nonwoven fabric laminate of any one of claims 1 to 3 through a hot melt adhesive.

5. The composite nonwoven fabric laminated sheet as of claim 4, which is a moisture-permeable nonwoven fabric laminated sheet obtained by laminating a moisture-permeable film on a surface of the first spunbonded nonwoven fabric through a hot melt adhesive.

6. The moisture-permeable nonwoven fabric laminated sheet as claimed in claim 5, wherein the moisture-permeable film is a porous film.

7. The composite nonwoven fabric laminated sheet as claimed in claim 4, which is obtained by laminating at least one sheet material selected from the group consisting of a nonwoven fabric, a woven fabric, a paper, a waterproof sheet, a net and a synthetic resin sheet on a surface of the first spunbonded nonwoven fabric of the nonwoven fabric laminate through a hot melt adhesive.

8. A sanitary product comprising the nonwoven fabric laminate of any one of claims 1 to 3 or the composite nonwoven fabric laminated sheet of any one of the claims 4 to 7.

9. The sanitary product of claim 8, which comprises the nonwoven fabric laminate of any one of claims 1 to 3 and the moisture-permeable nonwoven fabric laminated sheet of claim 5.

10. The sanitary product of claim 8, which is a disposable diaper having the moisture-permeable nonwoven fabric laminated sheet of claim 5 or 6 as a backsheet.

11. The sanitary product of claim 8, which is a sanitary napkin having the moisture-permeable nonwoven fabric laminated sheet of claim 5 or 6 as a backsheet.

12. A sanitary product, which comprises the composite nonwoven fabric laminated sheet of claim 7 as a solid gather.


**Patentansprüche**

1. Vliesstofflaminat, umfassend:

ein erstes Spinnvlies, das ein Polymer auf Propylenbasis umfasst,
ein Schmelzblas (Melt-Blown)-Vlies, das ein Polymer auf Propylenbasis umfasst und auf das erste Spinnvlies laminiert ist, und
ein zweites Spinnvlies, das ein Polymer auf Propylenbasis umfasst und auf das Melt-Blown-Vlies laminiert ist,
worin das erste Spinnvlies, das Melt-Blown-Vlies und das zweite Spinnvlies in dieser Reihenfolge laminiert sind;
das Vliesstofflaminat ein Gesamt-Basisgewicht von nicht mehr als 30 g/m$^2$ hat,
worin das Basisgewicht des ersten Spinnvlieses im Bereich von 3 bis 25 g/m$^2$ ist, das Basisgewicht des zweiten Spinnvlieses im Bereich von 1 bis 11 g/m$^2$ ist, das Verhältnis des Basisgewichts des ersten Spinnvlieses zum Basisgewicht des zweiten Spinnvlieses (Basisgewicht des ersten Spinnvlieses/Basisgewicht des zweiten Spinnvlieses) nicht weniger als 1,6 ist, das Verhältnis der Dicke des ersten Spinnvlieses zur Dicke des zweiten Spinnvlieses (Dicke des ersten Spinnvlieses/Dicke des zweiten Spinnvlieses) nicht weniger als 1,4 ist, das Basisgewicht des Melt-Blown-Vlieses weniger als 3 g/m$^2$ ist und das Kompressionsbindungs-Flächenverhältnis im Bereich von 6 bis 25 % ist.

**2.** Vliesstofflaminat gemäß Anspruch 1, worin die Fasern, die das erste Spinnvlies und das zweite Spinnvlies bilden, einen Faserdurchmesser von nicht mehr als 1,56 dtex (1,4 Denier) und die Fasern, die das Melt-Blown-Vlies bilden, einen Faserdurchmesser von 1 bis 5 $\mu$m haben.

**3.** Vliesstofflaminat gemäß Anspruch 1 oder 2, das einen 5 %-Modul-Index von 0,15 bis 0,38 N/30 mm/(g/m$^2$) hat.

**4.** Laminierte Verbund-Vliesstoffbahn, erhalten durch Laminieren mindestens eines Bahn-Materials, ausgewählt aus der Gruppe, bestehend aus Vliesstoff, Gewebestoff, Papier, einer wasserdichten Lage, einem Netz und einer Kunstharzlage oder einer feuchtigkeitsdurchlässigen Folie, auf eine Oberfläche des ersten Spinnvlieses des Vliesstofflaminats gemäß irgendeinem der Ansprüche 1 bis 3 durch einen Heißschmelzkleber.

**5.** Verbund-Vliesstoff-Laminatbahn gemäß Anspruch 4, die eine feuchtigkeitsdurchlässige Vliesstoff-Laminatbahn ist, erhalten durch Laminieren einer feuchtigkeitsdurchlässigen Folie auf eine Oberfläche des ersten Spinnvlieses durch einen Heißschmelzkleber.

**6.** Feuchtigkeitsdurchlässige Vliesstoff-Laminatbahn gemäß Anspruch 5, worin die feuchtigkeitsdurchlässige Folie eine poröse Folie ist.

**7.** Verbund-Vliesstoff-Laminatbahn gemäß Anspruch 4, die durch Laminieren mindestens eines Bahn-Materials, ausgewählt aus der Gruppe, bestehend aus Vliesstoff, Gewebestoff, Papier, einer wasserdichten Lage, einem Netz und einer Kunstharzlage, auf die Oberfläche des ersten Spinnvlieses des Vliesstofflaminats durch einen Heißschmelzkleber erhalten wird.

**8.** Hygieneartikel, der das Vliesstofflaminat gemäß irgendeinem der Ansprüche 1 bis 3 oder die Verbund-Vliesstoff-Laminatbahn gemäß irgendeinem der Ansprüche 4 bis 7 umfasst.

**9.** Hygieneartikel gemäß Anspruch 8, der das Vliesstofflaminat gemäß irgendeinem der Ansprüche 1 bis 3 und die feuchtigkeitsdurchlässige Vliesstoff-Laminatbahn gemäß Anspruch 5 umfasst.

**10.** Hygieneartikel gemäß Anspruch 8, der eine Wegwerf-Windel ist, die die feuchtigkeitsdurchlässige Vliesstoff-Laminatbahn gemäß Anspruch 5 oder 6 als Rückseitenlage umfasst.

**11.** Hygieneartikel gemäß Anspruch 8, der eine Damenbinde ist, die die feuchtigkeitsdurchlässige Vliesstoff-Laminatbahn gemäß Anspruch 5 oder 6 als Rückseitenlage umfasst.

**12.** Hygieneartikel, der die Verbund-Vliesstoff-Laminatbahn gemäß Anspruch 7 als Feststoffsammler umfasst.

**Revendications**

**1.** Stratifié de tissu non tissé, comprenant :

un premier tissu non tissé filé-lié comprenant un polymère à base de propylène,
un tissu non tissé fondu-soufflé comprenant un polymère à base de propylène et stratifié sur le premier tissu non tissé filé-lié, et
un second tissu non tissé filé-lié comprenant un polymère à base de propylène et stratifié sur le tissu non tissé fondu-soufflé,
dans lequel le premier tissu non tissé filé-lié, le tissu non tissé fondu-soufflé et le second tissu non tissé filé-lié sont stratifiés dans cet ordre ;
le stratifié de tissu non tissé possédant masse surfacique totale non supérieure à 30 g/m$^2$,
dans lequel la masse surfacique du premier tissu non tissé filé-lié est dans la plage de 3 à 25 g/m$^2$, la masse surfacique du second tissu non tissé filé-lié est dans la plage de 1 à 11 g/m$^2$, le rapport de la masse surfacique du premier tissu non tissé filé-lié par rapport à la masse surfacique du second tissu non tissé filé-lié (masse surfacique de premier tissu non tissé filé-lié/masse surfacique de second tissu non tissé filé-lié) n'est pas inférieur à 1,6, le rapport d'une épaisseur du premier tissu non tissé filé-lié par rapport à une épaisseur du second tissu non tissé filé-lié (épaisseur de premier tissu non tissé filé-lié/épaisseur de second tissu non tissé filé-lié) n'est pas inférieur à 1,4, la masse surfacique du tissu non tissé fondu-soufflé est inférieure à 3 g/m$^2$, et le rapport de superficie d'adhérence par compression est dans la plage de 6 à 25 %.

**2.** Stratifié de tissu non tissé selon la revendication 1, dans lequel des fibres pour former le premier tissu non tissé filé-lié et le second tissu non tissé filé-lié possèdent un diamètre des fibres non supérieur à 1,56 dtex (1,4 denier), et des fibres pour former le tissu non tissé fondu-soufflé possèdent un diamètre des fibres de 1 à 5 $\mu$m.

**3.** Stratifié de tissu non tissé selon la revendication 1 ou 2, qui possède un indice de module à 5 % de 0,15 à 0,38 N/30 mm/(g/m$^2$).

**4.** Feuille stratifiée de tissu non tissé composite obtenu en stratifiant au moins un matériau en feuille sélectionné parmi le groupe constitué d'un tissu non tissé, d'un tissu tissé, d'un papier, d'une feuille étanche à l'eau, d'un filet et d'une feuille de résine synthétique ou d'un film perméable à l'humidité sur une surface du premier tissu non tissé filé-lié du stratifié de tissu non tissé selon une quelconque des revendications 1 à 3 par l'intermédiaire d'un adhésif thermo-fusible.

**5.** Feuille stratifiée de tissu non tissé composite selon la revendication 4, qui est une feuille stratifiée de tissu non tissé perméable à l'humidité obtenu en stratifiant un film perméable à l'humidité sur une surface du premier tissu non tissé filé-lié par l'intermédiaire d'un adhésif thermo-fusible.

**6.** Feuille stratifiée de tissu non tissé perméable à l'humidité selon la revendication 5, dans laquelle le film perméable à l'humidité est un film poreux.

**7.** Feuille stratifiée de tissu non tissé composite selon la revendication 4, qui est obtenue en stratifiant au moins un matériau en feuille sélectionné parmi le groupe constitué d'un tissu non tissé, d'un tissu tissé, d'un papier, d'une feuille étanche à l'eau, d'un filet et d'une feuille de résine synthétique sur une surface du premier tissu non tissé filé-lié du stratifié de tissu non tissé par l'intermédiaire d'un adhésif thermo-fusible.

**8.** Produit sanitaire comprenant le stratifié de tissu non tissé selon une quelconque des revendications 1 à 3 ou la feuille stratifiée de tissu non tissé composite selon une quelconque des revendications 4 à 7.

**9.** Produit sanitaire selon la revendication 8, qui comprend le stratifié de tissu non tissé selon une quelconque des revendications 1 à 3 et la feuille stratifiée de tissu non tissé perméable à l'humidité selon la revendication 5.

**10.** Produit sanitaire selon la revendication 8, qui est une couche jetable possédant la feuille stratifiée de tissu non tissé perméable à l'humidité selon la revendication 5 ou 6 en tant que feuille de doublage.

**11.** Produit sanitaire selon la revendication 8, qui est une serviette hygiénique possédant la feuille stratifiée de tissu non tissé perméable à l'humidité selon la revendication 5 ou 6 en tant que feuille de doublage.

**12.** Produit sanitaire, qui comprend la feuille stratifiée de tissu non tissé composite selon la revendication 7 en tant que fronce solide.

[FIG. 1]

[FIG. 2]

[FIG. 3]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004003096 A

**Non-patent literature cited in the description**

- *JIS L 1092,* 1999
- *JIS L 1092,* 1992